# EUROPÄISCHE PATENTSCHRIFT

(11) **EP 2 150 609 B1**
(45) Veröffentlichungstag und Bekanntmachung des Hinweises auf die Patenterteilung: **17.11.2010**
(21) Anmeldenummer: 08760256.1
(22) Anmeldetag: 30.05.2008
(51) Int. Cl.: C12M 1/00, B01J 19/12, C12N 1/12

(54) **PHOTOREAKTOR**
PHOTOREACTOR
PHOTORÉACTEUR

(30) Priorität: 01.06.2007 DE 102007025748
(43) Veröffentlichungstag der Anmeldung: 10.02.2010
(73) Patentinhaber: Wacker Chemie AG, 81737 München (DE)
(72) Erfinder: DAUTH, Jochen, 84489 Burghausen (DE); VOIT, Harald, 84571 Reischach (DE)
(74) Vertreter: Schuderer, Michael
(86) Internationale Anmeldenummer: PCT/EP2008/056666
(87) Internationale Veröffentlichungsnummer: WO 2008/145719

(56) Entgegenhaltungen:
- WO-A-2006/013066
- WO-A-2007/047805
- DE-A1-102005 012 515
- JP-A- 10 098 964

## Beschreibung

Die Erfindung betrifft einen Photoreaktor mit LED-Silicon-Formteilen, gegebenenfalls in Kombination mit Lichtleiter-Formteilen als Strahlungsquelle; insbesondere Photobioreaktoren mit LED-Silicon-Formteilen, gegebenenfalls in Kombination mit Lichtleiter-Formteilen, als Strahlungsquelle.

Photoreaktoren, insbesondere Photobioreaktoren, werden zur großtechnischen Produktion von Mikroalgen beispielsweise Spirulina, Chlorella, Clamydomonas oder Haematococcus, von photosynthetischen Bakterien wie z.B. Cyanobakterien (beispielsweise Rhodobakter, Rhodospirillum), von Moosen oder anderen Pflanzenzellkulturen eingesetzt. Derartige Mikroalgen und Cyanobakterien sind in der Lage, mit Hilfe von Lichtenergie, CO₂ und Wasser in Biomasse umzuwandeln (Photosynthese). Dabei sind in der Regel zwei Pigmentkollektive beteiligt, wobei das erste Pigmentkollektiv eine gute Lichtabsorption bei Wellenlängen um 450 nm, das zweite Pigmentkollektiv eine gute Lichtabsorption bei einer Wellenlänge um 680 nm besitzt. Die Bildung von Biomasse erfolgt in einer Lichtreaktion und einer Dunkelreaktion. Die Lichtreaktion dient zur Umwandlung von Strahlungsenergie in chemische Energie bzw. Lichtquanten unter Bildung von Sauerstoff (= photochemische Wasserspaltung). Die Bildung der Biomasse (CH₂O)n erfolgt im 2. Schritt unter Verbrauch der Lichtquanten (Dunkelreaktion).

Photobioreaktoren werden zur Produktion von Algenbiomasse und z.B. Nahrungsmittel-, Nahrungsmittelergänzungsstoffen, Proteinen, Lipiden, Vitaminen, Antioxidantien, Wirkstoffen für Pharmazeutika und Kosmetika bis hin zu Öl aus Algen eingesetzt. CO₂ als Kohlenstoffquelle für die Algenkultivierung kann dabei als Luft, als mit CO₂ angereicherte Luft, als CO₂-haltiges Abgas oder als reines CO₂ in den Photobioreaktor zugeführt werden. Solche Photobioreaktoren können auch zur CO₂-Entfernung aus Abgasen eingesetzt werden.

Photobioreaktoren der ersten Generation nutzen das Sonnenlicht als Lichtquelle. Die Reaktoren bestehen aus großen offenen Beckenanlagen, z.B. Rundbeckenanlagen mit Durchmessern bis zu 45 m und umlaufenden Mischarmen. Nachteilig ist hierbei die Abhängigkeit von der Intensität der Sonneneinstrahlung und der Eintrag von Kontaminationen aufgrund des offenen Systems (www.ybsweb.co.jp: YAEYAMA Premium Quality Chlorella).

Die weltweit erste Photobioreaktoranlage zur Produktion von Mikroalgen in geschlossenen, sterilisierbaren Reaktoren wurde 2000 von der Firma Bisantech in Betrieb genommen (www.bisantech.de). Auch hier ist die Abhängigkeit von der Intensität der Sonneneinstrahlung als nachteilig zu benennen.

Ein geschlossener Photobioreaktor mit künstlicher Beleuchtung ist aus dem Patent US 6,602,703 B2 bekannt. Hier werden parallel angeordnete Leuchtröhren im Reaktor als Lichtquelle eingesetzt. Nachteilig ist hier der relativ hohe Energiebedarf und die Verschmutzungsneigung der Beleuchtungseinrichtung. Die US-Patentanmeldung US 2006/0035370 A1 beschreibt einen Mehrlagen-Photobioreaktor zur wachstumsgekoppelten Produktion eines nutzbringenden Metaboliten, bestehend aus einer ersten Kultivierungszone, die die Mikroorganismen und ein Kulturmedium für das vegetative Wachstum enthält und einer zweiten Kultivierungszone, die eine Seite der ersten Zone eng begrenzt und ein Kulturmedium und Mikroorganismen für die Produktion des Metaboliten enthält. Die zwei Kulturregionen sind voneinander durch eine transparente Trennwand getrennt. Als Lichtquelle wird Sonnenlicht oder künstliches Licht eingesetzt, mit den bereits oben genannten Nachteilen.

Aus der WO 92/00380 A1 ist bekannt, einen Photobioreaktor auszuleuchten, indem außerhalb des Reaktors eine Lichtquelle installiert wird und deren Licht über Lichtleiter in den Reaktor transportiert wird. Die Verwendung von LED-Leuchtkörpern (LED = Light Emitting Diode) oder auch Leuchtdioden genannten Leuchtkörpern als Strahlungsquelle für Photobioreaktoren ist bekannt: Die US 2005/0135104 A1 beschreibt den Einsatz von LEDs zur Beleuchtung von Kulturbehältern für marine Kulturen. Die LEDs sind dabei in einem transparenten Gehäuse eingeschlossen. Die Einbettung von LEDs in einer transparenten Kunststoffmatrix wird nicht beschrieben. Aus der JP 2007-040176 A ist bekannt die Stromversorgung für die künstliche Beleuchtung, beispielsweise mittels LEDs, von Reaktoren zur Algenkultivierung durch Windkraftwerke sicherzustellen. In der JP 2000-325073 A wird ein zweigeteilter Behälter zur Algenkultivierung beschrieben. Die Trennung der beiden Kompartimente des Behälters erfolgt mit einer Konstruktion, welche eine mit LEDs bestückte Leiterplatte umfaßt. Diese LED-bestückte Leiterplattte wird auf beiden Seiten mittels transparenter Platten vom Kulturmedium abgetrennt. Die JP 10-098964 A und die JP 11-089555 A beschreiben Algenreaktoren bei denen zur Beleuchtung LEDs eingesetzt werden. In einer Ausführungsform werden LED-Ketten in ein transparentes Rohr eingeführt und dieses zur Beleuchtung in den Reaktor abgesenkt. In der anderen Ausführungsform wird die LED-Kette vorher auf ein Substrat aufgeschweißt und dann in ein transparentes Rohr eingeführt. Die JP 2002-315569 A beschreibt ein Verfahren zur Algenproduktion, wobei LEDs zur Beleuchtung eingesetzt werden. Lange LED-Ketten werden dazu entweder zwischen Acrylglasplatten verlegt oder in transparente Rohre gehängt. Die Verwendung von LEDs als Lichtquelle für Bioreaktoren in einem geschlossenen Reaktor ist in der WO 2007/047805 A2 beschrieben. Nachteilig ist, dass die Beleuchtung mittels außenliegenden LEDs erfolgt. Die Durchstrahlung des Mediums ist deshalb unzureichend, erlaubt nur kurze Lichtweglängen von wenigen Zentimetern und erfordert für die Massenproduktion einen grossen Flächenbedarf.

Es bestand daher die Aufgabe, einen Photoreaktor zur Verfügung zu stellen, der die Kultivierung in einem abgeschlossenen System und mit einer möglichst gleichmäßigen und optimalen Strahlungsintensität über das gesamte Volumen ermöglicht. Eine weitere Aufgabe bestand in der Erhöhung der Produktivität von Photosynthesereaktoren, um die Produktion in großtechnischem Maßstab zu optimieren. Darüberhinaus sollten die LEDs in Gestalt solcher Formteile zur Verfügung gestellt werden, welche den unterschiedlichsten Umwelteinflüssen, seien es physikalische, chemische oder biologische, über einen langen Zeitraum wirksam standhalten. Eine weitere Aufgabe bestand darin, nicht nur die einzelnen LEDs, sondern auch die elektrischen Verbindungen zwischen den LEDs und deren Anschlüsse an Stromquellen wirksam vor solchen Umwelteinflüßen zu schützen.

Gegenstand der Erfindung sind Photoreaktoren mit LED-Kunststoff-Formteilen, in denen ein oder mehrere LED-Leuchtkörper in einer Kunststoffmatrix eingeschlossen sind, als Strahlungsquellen, welche im Innern des Photoreaktors angeordnet sind, dadurch gekennzeichnet, dass als LED-kunstoff-Formteilen LED-Silicon-Formteile eingesetzt werden.
Als Photoreaktoren werden geschlossene Reaktoren bevorzugt. Die Reaktoren sind mit einem drucktragenden, gegebenenfalls temperaturbeständigem Mantel, beispielsweise aus Stahl, Edelstahl, Kunststoff, Emaille, oder Keramik gefertigt. Es können transparente und nicht transparente Materialien eingesetzt werden, bevorzugt werden bei geschlossenen Reaktoren nicht transparente. Die Reaktorvolumina können beliebig gewählt werden. Im Unterschied zum Stand der Technik ist bei geschlossenen Reaktoren keine Beschränkung auf geringe Volumina notwendig, da mit den innen angeordneten LED-Silicon-Formteilen als Strahlungsquellen eine gleichmäßige Beleuchtung des Innenraumes erhalten wird, und somit die Reaktorabmessungen vom Lichtweg entkoppelt werden.

Aufgrund der drucktragenden Materialien ist im Gegensatz zu Reaktoren, welche aus Glas oder transparentem Kunststoff bestehen, eine hohe, grundflächensparende Bauweise für die Massenproduktion möglich. Die Anordnung der LED-Silicon-Formteile zu Lichteinbauten in solchen Reaktoren ermöglicht die Kombination von mehreren Reaktorsegmenten zu hohen kolonnenartigen Reaktoren mit entsprechender Platzersparnis, das heißt enorm hohe Grundflächenproduktivität. Weiter kann bei erhöhtem Druck gearbeitet werden, vorzugsweise in einem Bereich von 0,1 bis 5 bar Überdruck. Bei Photobioreaktoren bedingt ein höherer Druck einen höheren CO₂-Partialdruck in dem CO₂-haltigen Gas mit dem der Reaktor beschickt wird, was zu einer höheren CO₂-Gleichgewichtskonzentration im Medium führt, den CO₂-Transport in die Mikroalgen beschleunigt und eine Intensivierung der Photosynthese-Reaktion bedingt. Wird als CO₂-Quelle CO₂-haltiges Abgas eingesetzt, wird die Gleichgewichtskonzentration im Medium ebenso/zusätzlich erhöht, und dadurch Photosynthese und Algenwachstum deutlich beschleunigt.

Durch die hohe, grundflächensparende Bauweise des Photobioreaktors wird eine längere Gasverweilzeit eingestellt (die Aufstiegsgeschwindigkeit der Gasblasen bleibt gleich) und dadurch eine wesentlich bessere CO₂-Abreicherung erreicht. Neben der besseren CO₂-Abreicherung kann mit solchen Anlagen auch eine höhere Anreicherung von Gasen im Abgas des Photobioreaktors erhalten werden, wodurch z.B. die Gewinnung von Sauerstoff oder auch Wasserstoff, bei Wasserstoff erzeugenden Algen, wirtschaftlich möglich wird. Darüberhinaus reduziert bzw. verhindert ein geschlossener Reaktor den Verdunstungsverlust bei Wasser, was insbesondere in wasserarmen (ariden) Regionen der Erde vorteilhaft ist.

Beim Einsatz von LED-Silicon-Formteilen, gegebenenfalls in Kombination mit Lichtleiter-Formteilen als Strahlungsquellen, welche im Innern des Photoreaktors angeordnet sind, besteht auch keine Abhängigkeit mehr von Sonnenlicht.

Durch die druck- und temperaturfeste Ausführung des Reaktormantels in Verbindung mit temperaturbeständigen LED-Kunststoff-Formteilen kann gegebenenfalls auch bei höherer Temperatur gearbeitet werden. Eine Sterilisation des Reaktors mit Dampf bei 120°C ist möglich und damit die wirksame Vermeidung von Kontaminationen sowie auch der Einsatz genmanipulierter Mikroalgen oder Cyanobakterien. Die Reinigung des Reaktors kann ebenso sehr effektiv durch CIP ("Cleaning in place")-Dampfstrahlen bei 120°C erfolgen.

Der Reaktor ist zur Befüllung und Nährstoffversorgung mit Zuleitungen und zur Produktabtrennung und Entleerung mit Ableitungen ausgestattet. Für eine kontinuierliche Fahrweise empfiehlt sich gegebenenfalls die Ausstattung mit einem externen Loop, in dem Phasentrennapparate oder Module zur Dialyse, Umkehrosmose sowie Mikro- oder Nanofiltration angeordnet sind. Zur Wärmeabfuhr und Beheizung kann der Reaktor gegebenenfalls mit einem Doppelmantel, Halbrohrschlangen an den Reaktorwänden oder innenliegenden Wärmeaustauschern ausgestattet werden. Des weiteren kann der Reaktor noch Rühreinrichtungen und Pumpen zur Durchmischung enthalten. Vorzugsweise erfolgt die Durchmischung durch Begasen mit dem Feed-Gas analog in einer Blasensäule nach dem Airlift-Prinzip, ohne zusätzliche mechanische Energie. Blasensäulen/Airlift-Reaktoren werden mit einer Gasgeschwindigkeit von vorzugsweise 0,005 bis 2,0 m/s betrieben, besonders bevorzugt in einem Bereich von 0,02 bis 0,2 m/s. Vorzugsweise wird der Reaktor in mehrere Reaktoreinheiten unterteilt und die Reaktoreinheiten übereinander, bevorzugt versetzt, gestapelt angeordnet, wobei die Reaktoreinheiten über Durchtrittsöffnungen miteinander verbunden sind. Die Reaktorzellen können dabei durch Flansche (analog Kolonnenschüsse) miteinander verbunden werden oder oder bevorzugt durch Einbauten (analog Kolonnenböden) in einem gemeinsamen Außenmantel gebildet werden.

Beim Betrieb als Photobioreaktor liegt beispielsweise bei einer Algenwachstumsrate von 0,025 1/h, einer Algenkonzentration von 2,5 Gew.-% im wässrigen Reaktormedium, und einer Gasgeschwindigkeit von 0,05 m/s, bei der Verwendung eines typischem Kraftwerksabgases als CO₂-Quelle, die optimale Reaktorhöhe bei etwa 20 m.

Als Strahlungsquelle werden LED-Silicon-Formteile welche ein oder mehrere LED-Leuchtkörper in einer oder mehreren Kunststoff-Matrices enthalten, eingesetzt. Geeignete LED-Leuchtkörper sind strahlungsemittierende Halbleiterbauelemente von organischen oder anorganischen Halbleitern, sogenannte LEDs. Bei den LEDs kann es sich um bereits mit Kunststoff, meist Silicon, verkapselte Dioden oder um unverkapselte Dioden handeln. Die LEDs können im InfrarotBereich, im sichtbaren Bereich oder im UV-Bereich strahlen. Die Auswahl hängt von der beabsichtigten Anwendungen ab. Für die Photosynthese in Photobioreaktoren werden LEDs bevorzugt, welche im sichtbaren Bereich strahlen, insbesondere rotes Licht. Die in der Kunststoff-Matrix eingebetteten LED-Leuchtkörper können bei gleicher Wellenlänge emittieren. Es können aber auch LED-Leuchtkörper mit unterschiedlicher Strahlungscharakteristik miteinander kombiniert werden. Im allgemeinen sind mehrere LED-Leuchtkörper miteinander leitend verbunden, in Reihe und/oder parallel geschaltet. Die Leuchtkörperanordnung kann mit Sensoren verbunden sein, sowie mit Mess/Steuerungseinrichtungen. Die Anzahl der Leuchtkörper und deren Anordnung zueinander hängt von deren Anwendung ab. Die LED-Leuchtkörper können kontinuierlich oder gepulst betrieben werden. Die Stromversorgung ist beliebig, vorzugsweise erfolgt sie mittels Solarzellen.

Ein Lichtleiter ist eine Faser aus einem transparenten, lichtdurchlässigen Material, meist Glas oder Kunststoff, die dem Transport von Licht oder Infrarotstrahlung dient. Beispiele für Lichtleiter sind Lichtwellenleiter, Glasfasern, polymere optische Fasern oder andere lichtleitende Bauteile aus Kunststoff sowie Faseroptik-Komponenten. Die Lichtleiter sind so ausgerüstet, dass das Licht gleichmäßig über die gesamte Ausdehnung des Lichtleiters abgegeben wird. Gegebenenfalls können die Lichtleiter mit einer Linse ausgerüstet werden, um das Licht vor Einleitung in den Lichtleiter zu bündeln und zu verstärken. Der Einsatz von Lichtleiter-Formteilen als Strahlungsquelle neben Kunststoff-LED-Formteilen ist beim Betrieb am Tag vorteilhaft. Bevorzugt werden Lichtleiter auf der Basis von Thermoplastischen Silicon-Elastomeren (TPSE). Thermoplastische Siliconelastomere enthalten einen Organopolymeranteil, beispielsweise Polyurethan oder Polyvinylester, und einen Siliconanteil, meist auf Basis von Polydialkylsiloxan-Basis der obengenannten Spezifikation. Geeignete Thermoplastische Siliconelastomere sind im Handel erhältlich, beispielsweise die entsprechenden Geniomer^{R}-Typen der Wacker Chemie.

Die Formgestalt der LED-Silicon-Formteile und der Lichtleiter ist beliebig. Sie können in Form von Schläuchen vorliegen, als Bänder, als Rohre, als Platten oder in Form von Matten.

Platten, Matten oder Bänder werden, insbesondere bei LED-Silicon -Formteilen als Strahlungsquelle, vorzugsweise dazu eingesetzt um die Innenwände des Reaktors mit Strahlungsquellen auszustatten. Die Ausgestaltung in Form von Rohren oder Schläuchen bietet sich für den Einbau von Strahlungsquellen im Reaktorinnenraum an.

Zur gleichmäßigen Versorgung mit Licht im Innenraum großer Reaktoren können mehrere LED-Silicon-Formteile, die von der Algensuspension umströmt werden und beispielsweise als Rohre, Schläuche oder Platten ausgebildet sind, als Rohr-, Schlauch- oder Plattenbündel zu Lichteinbauten zusammengefasst werden. Die Leuchtkörper können in einer besonders bevorzugten Ausführungsform in einer solchen Weise zueinander versetzt angeordnet werden (z.B. verdrillt), dass sie den Umgebungsraum vollständig und homogen ausleuchten.

Bei einem Rohrbündel, bestehend aus LED-Silicon-Formteilen, beträgt der erforderliche Abstand zwischen benachbarten Formteiloberflächen und einer etwa der natürlichen Sonneneinstrahlung entsprechenden Lichtemission, bezogen auf die Formteiloberfläche, etwa 1 bis 10 cm, bevorzugt 2 bis 5 cm.

Ein Photobioreaktor kann auch LED-Silicon-Formteile in Röhren- oder Plattenform enthalten, die von der Algensuspension durchströmt werden. Dabei können mehrere Rohre oder Platten zu Rohr- oder Plattenbündel zusammengefasst werden und eine Reaktoreinheit bilden, die im Mantelraum durch ein Kühlmedium durchströmt und dadurch gekühlt wird. Die Dimensionierung der LED-Silicon-Formteile oder der Lichtleiter-Formteile ist beliebig und kann der Reaktorgröße angepasst werden.

Der Abstand zwischen den LED-Silicon-Formteilen und/oder den Lichtleiterformteilen kann durch unterstützende Lichtverteilung im Inneren des Photoreaktors durch "lumineszierende Silicon-Schwebekörper" mit eingestellter optimaler Wellenlänge und minimaler Dichtedifferenz erhöht werden, die vorzugsweise allein durch die Begasung suspendiert werden. Diese "lumineszierenden Silicon-Schwebekörper" bestehen aus einem oder mehreren lumineszierenden Stoffen in einer Siliconmatrix aus beispielsweise thermoplastischen Siliconelastomeren wie in EP 1412416 B1 oder EP 1489129 B1 beschrieben.

Die Materialien für die LED-Kunststoff-Formteile sind Silicone.

In den LED-Silicon-Formteilen werden die LEDs vollständig in die Kunstoffmatrix eingebettet und von dieser umhüllt, vorzugsweise einschließlich der elektrisch leitenden Verbindungen zwischen den LEDs, und besonders bevorzugt auch die Anschlüsse der LED-Ketten an die Stromversorgung.

Beispiele für geeignete Silicone sind vernetzte Siliconkautschuke, welche durch Kondensations- oder Additionsreaktion oder radikalisch vernetzen. Die Vernetzungsreaktion kann kationisch, mittels entsprechender Katalysatoren, oder radikalisch, mittels Peroxiden, oder durch Strahlung, insbesondere UV-Strahlung, oder thermisch initiert werden. Systeme, welche zu vernetzten Siliconkautschuken führen, sind vorzugsweise als 1-oder 2-, aber auch als Mehrkomponenten-Systeme im Handel erhältlich. Geeignet sind auch Siliconhybridpolymere und Siliconharze. Die Schichtdicke der Siliconmatrix hängt von der Anwendung der LED-Silicon-Formteile ab, und beträgt im allgemeinen zwischen 0,1 und 50 mm.

Kondensationsvernetzende Siliconkautschuk-Systeme enthalten
a) kondensationsfähige Endgruppen aufweisende Organopolysiloxane,
b) gegebenenfalls je Molekül mindestens drei an Silicium gebundene hydrolysierbare Gruppen aufweisende Organosiliciumverbindungen, sowie
c) Kondensationskatalysatoren.

Geeignete vernetzte Siliconkautschuke, welche durch Kondensationsreaktion vernetzen, sind bei Raumtemperatur vernetzende 1-Komponenten-Systeme, sogenannte RTV-1-Siliconkautschuke. Bei den RTV-1-Siliconkautschuken handelt es sich um Organopolysiloxane mit kondensationsfähigen Endgruppen, welche in Gegenwart von Katalysatoren unter Kondensation bei Raumtemperatur vernetzen. Am gebräuchlichsten sind Dialkylpolysiloxane der Struktur R₃SiO[-SiR₂O]ₙ-SiR₃ mit einer Kettenlänge von n > 2. Die Alkylreste R können gleich oder verschieden sein und haben im allgemeinen 1 bis 4 C-Atome und können gegebenenfalls substituiert sein. Die Alkylreste R können auch teilweise durch andere Reste ersetzt sein, vorzugsweise durch Arylreste, welche gegebenenfalls substituiert sind, und wobei die Alkyl(Aryl)-Gruppen R teilweise durch zur Kondensations-Vernetzung fähige Gruppen ausgetauscht sind, beispielsweise Alkohol-, Acetat-, Amin- oder Oximreste. Die Vernetzung wird mittels geeigneter Katalysatoren, beispielsweise Zinn- oder Titankatalysatoren katalysiert. Geeignete RTV-1-Siliconkautschuke sind im Handel erhältlich, beispielsweise die entsprechenden Typen der ELASTOSIL® A-, E- oder N-Reihe der Wacker Chemie AG.

Geeignete vernetzte Siliconkautschuke, welche durch Kondensationsreaktion vernetzen, sind bei Raumtemperatur vernetzende 2-Komponenten-Systeme, sogenannte RTV-2-Silikonkautschuke. RTV-2-Siliconkautschuke erhält man mittels Kondensationsvernetzung von mehrfach mit Hydroxygruppen substituierten Organopolysiloxanen in Gegenwart von Kieselsäureestern. Als Vernetzer können auch Alkylsilane mit Alkoxy-, Oxim- Amin- oder Acetatgruppen eingesetzt werden, welche in Anwesenheit von geeigneten Kondensations-Katalysatoren, beispielsweise Zinn- oder Titankatalysatoren mit den Hydroxygruppen-terminierten Polydialkylsiloxanen vernetzen. Geeignete kondensationsvernetzende RTV-2-Siliconkautschuke sind im Handel erhältlich, beispielsweise die entsprechenden Typen der ELASTOSIL® RT-Reihe der Wacker Chemie AG.

Beispiele für die in RTV-1- und RTV-2-Siliconkautschuk enthaltenen Polydialkylsiloxane sind solche der Formel (OH)R₂SiO[-SiR₂O]ₙ-SiR₂(OH) mit einer Kettenlänge von n > 2, wobei die Alkylreste R gleich oder verschieden sein können, im allgemeinen 1 bis 4 C-Atome enthalten und gegebenenfalls substituiert sein können. Die Alkylreste R können auch teilweise durch andere Reste ersetzt sein, vorzugsweise durch Arylreste, welche gegebenenfalls substituiert sind. Vorzugsweise enthalten die Polydialkylsiloxane terminale OH-Gruppen, welche mit den Kieselsäureestern bzw. dem System Alkylsilan/Zinn(Titan)katalysator bei Raumtemperatur vernetzen.

Beispiele für die in RTV-1- und RTV-2-Siliconkautschuken enthaltenen, hydrolysierbare Gruppen aufweisende Alkylsilane sind solche der Formel RₐSi(OX)₄₋ₐ, mit a = 1 bis 3 (bevorzugt 1), und X in der Bedeutung von R" (Alkoxyvernetzer), C(O)R" (Acetatvernetzer), N=CR"₂ (Oxim-Vernetzer) oder NR"₂ (Aminvernetzer), wobei R" einen einwertigen Kohlenwasserstoffrest mit 1 bis 6 Kohlenstoffatomen bedeutet.

Additionsvernetzende Siliconkautschuk-Systeme enthalten
a) Organosiliciumverbindungen, die Reste mit aliphatischen Kohlenstoff-Kohlenstoff-Mehrfachbindungen aufweisen,
b) gegebenenfalls Organosiliciumverbindungen mit Si-gebundenen Wasserstoffatomen oder anstelle von a) und b)
c) Organosiliciumverbindungen, die Reste mit aliphatischen Kohlenstoff-Kohlenstoff-Mehrfachbindungen und Si-gebundene Wasserstoffatome aufweisen,
d) die Anlagerung von Si-gebundenen Wasserstoff an aliphatische Mehrfachbindung fördernde Katalysatoren und
e) gegebenenfalls die Anlagerung von Si-gebundenem Wasserstoff an aliphatische Mehrfachbindung bei Raumtemperatur verzögernde Mittel.

Geeignete vernetzte Siliconkautschuke, welche durch Additionsreaktion vernetzen, sind bei Raumtemperatur vernetzende 2-Komponenten-Systeme, sogenannte additionsvernetzende RTV-2-Silikonkautschuke. Additionsvernetzende RTV-2-Siliconkautschuke erhält man durch mit Pt-Katalysatoren katalysierte Vernetzung von mehrfach ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten aus Dialkylpolysiloxanen der Struktur R₃SiO[-SiR₂O]ₙ-SiR₃ mit n ≥ 0, im allgemeinen mit 1 bis 4 C-Atomen im Alkylrest, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur CH₂=CH₂-R₂SiO[-SiR₂O]ₙ-SiR₂-CH₂=CH₂ eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden.

Als Vernetzungskatalysator sind geringe Mengen einer platinorganischen Verbindung enthalten.

Geeignete RTV-Siliconkautschuke sind im Handel erhältlich, beispielsweise die entsprechenden Typen der ELASTOSIL® RT oder ELASTOSIL® LR (LSR-Siliconkautschuk) oder SEMICOSIL®-Reihe der Wacker Chemie AG.

Geeignete radikalisch oder durch Additionsreaktion vernetzende Siliconkautschuke sind die bei Temperaturerhöhung vernetzenden Festsiliconkautschuke (HTV).

Additionsvernetzende HTV-Siliconkautschuke erhält man durch die Vernetzung von mehrfach ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten der peroxidisch oder additionsvernetzenden HTV-Siliconkautschuke aus Dialkylpolysiloxanen der Struktur R₃SiO[-SiR₂O]ₙ-SiR₃ mit n ≥ 0, im allgemeinen mit 1 bis 4 C-Atomen im Alkylrest, wobei die Alkylreste ganz oder teilweise durch Arylreste wie den Phenylrest ersetzt werden können und an einem oder an beiden Enden einer der endständigen Reste R durch eine polymerisierbare Gruppe wie die Vinylgruppe ersetzt ist. Es können aber auch Polymere mit seitenständigen bzw. mit seiten- und endständigen Vinylgruppen verwendet werden. Bevorzugt werden vinylendblockierte Polydimethylsiloxane der Struktur CH₂=CH₂-R₂SiO[-SiR₂O]ₙ-SiR₂-CH₂=CH₂ eingesetzt, sowie vinylendblockierte Polydimethylsiloxane der genannten Struktur, welche noch seitenständige Vinylgruppen tragen. Bei additionsvernetzenden HTV-Siliconkautschuken ist die zweite Komponente ein Copolymer aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysatoren kommen Platinkatalysatoren zum Einsatz

HTV-Siliconkautschuke werden auch als Einkomponentensystem verarbeitet, wobei die Vernetzungsreaktion durch Temperaturerhöhung und in Anwesenheit von Peroxiden als Vernetzungskatalysatoren wie z.B. Acyl-, Alkyl- oder Arylperoxiden induziert wird. Peroxidvernetzende HTV-Siliconkautschuke erhält man durch die Vernetzung von gegebenenfalls mehrfach mit ethylenisch ungesättigten Gruppen, vorzugsweise Vinylgruppen, substituierten Organopolysiloxanen. Geeignete HTV-Siliconkautschuke sind im Handel erhältlich, beispielsweise die entsprechenden ELASTOSIL® R- oder ELASTOSIL® R plus-Typen der Wacker Chemie AG.

In jüngster Zeit sind zudem noch spezielle HTV- und RTV-1-Siliconkautschuke im Handel erhältlich, welche über die beschriebene Additionsreaktion vernetzt werden, indem spezielle Platin-Komplexe bzw. Platin-/Inhibitor-Systeme thermisch und/oder photochemisch aktiviert werden und somit die Vernetzungsreaktion katalysieren. Derartige Systeme sind beispielsweise als ELASTOSIL® R-Typen, ELASTOSIL® RT-Typen und Semicosil®-Typen bei der Wacker Chemie AG erhältlich.

Geeignete Materialien sind auch Siliconhybridpolymere. Siliconhybridpolymere sind Copolymerisate oder Pfropfcopolymerisate von Organopolymerblöcken, beispielsweise Polyurethan, Polyharnstoff oder Polyvinylestern, und Siliconblöcken, im allgemeinen auf Basis von Polydialkylsiloxanen der obengenannten Spezifikation. Beispielsweise werden thermoplastische Siliconhybridpolymere in EP 1412416 B1 und EP 1489129 B1 beschrieben, deren diesbezügliche Offenbarung auch Gegenstand dieser Anmeldung sein soll. Derartige Siliconhybridpolymere werden als Thermoplastische Siliconelastomere (TPSE) bezeichnet und sind im Handel erhältlich, beispielsweise die entsprechenden GENIOMER®-Typen der Wacker Chemie AG.

Siliconharze sind ebenfalls geeignete Materialien für die Siliconmatrix. Im allgemeinen enthalten die Siliconharze Einheiten der allgemeinen Formel R_{b}(RO)_{c}SiO_{(4-b-c)/2}, worin
b gleich 0, 1, 2 oder 3 ist,
c gleich 0, 1, 2 oder 3 ist,
mit der Maßgabe, dass b+c ≤ 3 ist,
und R in der oben dafür angegebenen Bedeutung, welche ein hoch vernetztes Organosilicon-Netzwerk aufbauen. Geeignete Siliconharze sind im Handel erhältlich, beispielsweise die entsprechenden SILRES®-Typen der Wacker Chemie AG.

Geeignet sind auch strahlungshärtende Acryl-, Epoxy- oder Vinylether-funktionelle Silicone, die mit Radikalbildnern oder kationischen Photoinitiatoren ausgehärtet werden.

Sind die LED-Leuchtkörper in einer einzelnen Siliconmatrix eingebettet, muss hierbei die Siliconmatrix vollständig und dauerhaft an den LED-Leuchtkörper über die gesamte Betriebsdauer des Reaktors angebunden sein, damit die Lichtleistung während der Betriebsdauer des Photoreaktors nicht abnimmt. Besonders geeignet sind hierbei Silicone, die sich an den Leuchtkörper in ihrer Form anpassen, gut haften und keine Hohlräume zwischen Matrix und Leuchtkörper aufgrund z.B. erfolgender Temperaturschwankungen bilden. Bevorzugte Materialien sind die genannten RTV-2-Siliconkautschuke, insbesondere LSR-Siliconkautschuk, HTV-Siliconkautschuke und die Siliconhybridpolymere, insbesondere thermoplastische Elastomere wie die weiter oben beschrieben.

In einer bevorzugten Ausführungsform enthalten die LED-Silicon-Formteile eine innere, weiche Siliconmatrix A, welche von einer oder mehreren, härteren Siliconmatrices B umgeben wird. Die innere Siliconmatrix A ist weich mit einer Shore Härte A (DIN 53 505 / ISO 868) von kleiner oder gleich 10, oder, falls es sich um ein flüssiges Siliconöl handelt, einer durchschnittlichen Viskosität (bei 23 °C und 1013 mbar) von 1 bis 100x10⁶ mPa.s. Vorzugsweise beträgt die Shore-Härte A unter 5 bzw. die durchschnittliche Viskosität (bei 23 °C und 1013 mbar) von 10 bis 10x10⁶ mPa.s. Bei der äußeren Siliconmatrix B beträgt die Shore-Härte A mehr als 10, und für den Fall, dass die innere Siliconmatrix A, ebenfalls aus einem bei Normalbedingungen (23/50 DIN 50014) festen Silicontyp besteht, die Differenz zwischen den Shore-Härten A von innerer Siliconmatrix A und äußerer Siliconmatrix B mindestens 5 Shore-Härte-Punkte, bevorzugt mindestens 10, insbesondere mindestens 20 beträgt.

Die äußere Siliconmatrix B basiert auf den obengenannten Materialien.

Die Siliconmatrix A ist auf den Leuchtkörper hin optimiert und soll neben der Schutzfunktion für die elektronischen Bauteile (Schockabsorption), die Lichtausbeute optimieren (Brechungsindex-Anpassung) und die Wärmeabfuhr erleichtern. Weiter ist von Bedeutung, dass die Leuchtkörper dauerhaft, während der gesamten Betriebszeit der Leuchtkörper, von der Siliconmatrix fest umschlossen bleiben, und Luft- und Wassereinschlüsse, die zu diffusen Lichtstreuungseffekten führen, verhindert werden.

Bevorzugte Materialien für die innere Siliconmatrix A sind Siliconöle, das sind im allgemeinen Dialkylpolysiloxane der Struktur R₃SiO[-SiR₂O]ₙ-SiR₃ mit einer Kettenlänge von n > 2. Die Alkylreste R können gleich oder verschieden sein und haben im allgemeinen 1 bis 4 C-Atome und können gegebenenfalls substituiert sein. Die Alkylreste R können auch teilweise durch andere Reste ersetzt sein, vorzugsweise durch Arylreste, welche gegebenenfalls substituiert sind, oder durch Trialkylsiloxy-Gruppen bei verzweigten Siliconölen. Beispiele sind Methylsiliconöle (CH₃)₃SiO[-Si(CH₃)₂O]ₙ-Si(CH₃)₃, Methylphenylsiliconöle (CH₃)₃SiO[-Si(CH₃)₂O]_{n'}-[-Si(C₆H₅)₂O]_{n"}-Si(CH₃)₃ oder (CH₃)₃SiO[-Si(CH₃)₂O]_{n'}-[-Si(CH₃) (C₆H₅)O]_{n"}-Si(CH₃)₃ mit jeweils n'+ n" > 2, verzweigte Methylsiliconöle
(CH₃)₃SiO[-Si(CH₃)(OSi(CH₃)₃)O]ₙ-Si(CH₃)₃, verzweigte Methylphenylsiliconöle (CH₃)₃SiO[-Si(C₆H₅)(OSi(CH₃)₃)O]ₙ-Si(CH₃)₃. Mit der Einführung von Arylgruppen und der Einstellung des Verhältnisses von Alkyl- zu Aryl-Gruppen kann der Fachmann in bekannter Weise den Brechungsindex der Siliconmatrix dem Leuchtkörper anpassen. Desweiteren können auch vorzugsweise an den Endgruppen funktionalisierte ("nicht-gestopperte") Polydimethylsiloxanöle verwendet werden. Solche Siliconöle sind im Handel erhältlich und mit bekannten Methoden herstellbar. Beispiele für kommerziell erhältliche Siliconöle sind die Wacker Siliconöle der Wacker Chemie AG.

Für die innere Siliconmatrix A geeignet sind auch Silicongele. Silicongele werden aus zwei gießbaren Komponenten hergestellt, welche bei Raumtemperatur in Gegenwart eines Katalysators vernetzen. Eine der Komponenten besteht im allgemeinen aus Dialkylpolysiloxanen der Struktur R₃SiO[-SiR₂O]ₙ-SiR₃ mit n ≥ 0, im allgemeinen mit 1 bis 4 C-Atomen im Alkylrest, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur CH₂=CH₂-R₂SiO[-SiR₂O]ₙ-SiR₂-CH₂=CH₂ eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysator sind geringe Mengen einer platinorganischen Verbindung enthalten. Durch das Mischen der Komponenten wird die Vernetzungsreaktion ausgelöst und das Gel gebildet. Diese Vernetzungsreaktion kann durch das Einwirken von Wärme und/oder durch elektromagnetische Strahlung, vorzugsweise UV-Strahlung, beschleunigt werden. UV-LEDs selbst können dabei die Vernetzungsreaktion von Gelen induzieren. Bei den Silicongelen handelt es sich um besonders weiche Materialien, besonders bevorzugt mit einer Shore-Härte 00 von unter 50 (DIN 53 505 / ISO 868), am meisten bevorzugt um solche mit einem Penetrationswert gemäß DIN ISO 2137 von > 10 mm/10 (bei 9,38 g Viertelkonus und 5 s Einwirkzeit). Geeignete Silicongele sind im Handel erhältlich, beispielsweise unter dem Handelsnamen WACKER SilGel® der Wacker Chemie AG, München.

Die Herstellung der LED-Kunststoff-Formteile kann mittels in der Kunststoffverarbeitung üblichen Technologien erfolgen, beispielsweise mittels Vergießen, Extrusion, Cast-Molding, Compression Molding oder Spritzguss erfolgen, je nach Gestalt des LED-Kunststoff-Formteils. Die LED-Silicon-Formteile können gegebenenfalls noch mit einem Topcoat beschichtet werden, beispielsweise auf Siliconharzbasis.

Durch eine optimierte konstruktive Anordnung einer großen Anzahl von LED-Kunststoff-Formteilen im Innenraum großer Fermenter, im Sinne von Reaktoreinbauten, wird eine wirtschaftliche Massenproduktion von Algenbiomasse in großvolumigen Fermentern ermöglicht. Mit den LED-Kunststoff-Formteilen kann eine Vielzahl von LEDs zu Reaktorleuchteinbauten zusammengefasst werden, die somit eine optimale Innenbeleuchtung eines großen Reaktorvolumens wirtschaftlich ermöglichen.

Aufgrund der Einbettung der LEDs in eine Kunststoffmatrix werden, insbesondere im Falle einer Siliconmatrix Fouling und Belagsbildung deutlich reduziert. Ein weiterer Vorteil besteht darin, dass durch die Verwendung von LED-Silicon-Formteilen eine wiederkehrende Dampfsterilisation des gesamten Photobioreaktors bei mindestens 121°C über einen Zeitraum von wenigstens 1 Stunde ermöglicht wird.

Durch die Anordnung der LED-Kunststoff-Formteile über das gesamte Reaktorvolumen wird eine bessere Wärmeabfuhr erzielt, wodurch die LEDs bei höherer Spannung und Lichtausbeute betrieben werden können, als dies bei einer Außenanordnung möglich ist.

Der Photoreaktor kann als Reaktor für strahleninduzierte chemische Reaktionen eingesetzt werden. Der Photoreaktor kann als Photobioreaktor zur autotrophen und heterotrophen Produktion von Wertstoffen wie Proteinen, Vitaminen, pharmazeutischen Wirkstoffen, Lipiden, sowie Wasserstoff aus CO₂, Wasser und mineralischen Nährstoffen, mittels Mikroalgen eingesetzt werden. Weitere Anwendungen sind die mixotrophe Produktion von Wertstoffen unter Zufütterung organischer Kohlenstoffquellen. Die Produktion von Algenbiomasse aus CO₂ und Wasser und mineralischen Nährstoffen, und die Entfernung von CO₂ aus Kraftwerks- oder Industrieabgasen. Die so hergestellt Algenbiomasse eignet sich als Energierohstoff, Chemierohstoff, Nahrungsmittel, und zum Einsatz in kosmetischen und medizinischen Anwendungen. Weiter ist der Reaktor geeignet für Verfahren zur Produktion von Wasserstoff oder anderen gasförmigen Stoffwechselprodukten mit Mikroalgen oder Mikroorganismen, die eine Energiezufuhr in Form von Licht erfordern.

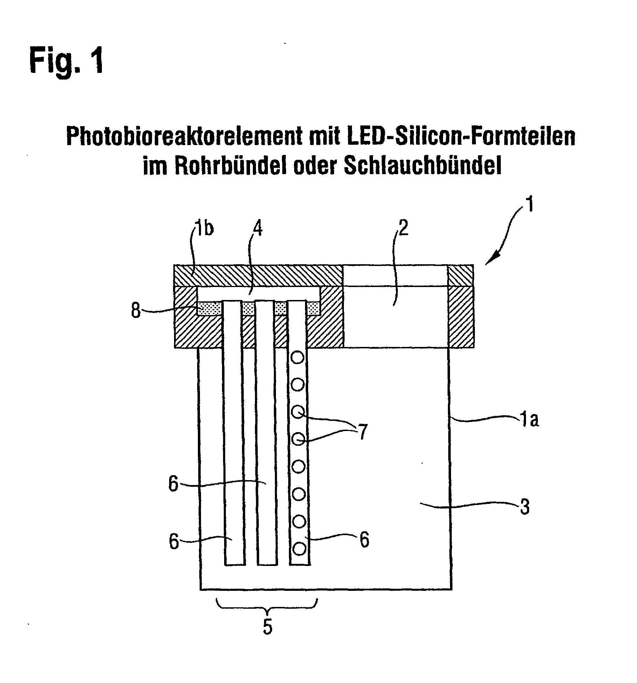

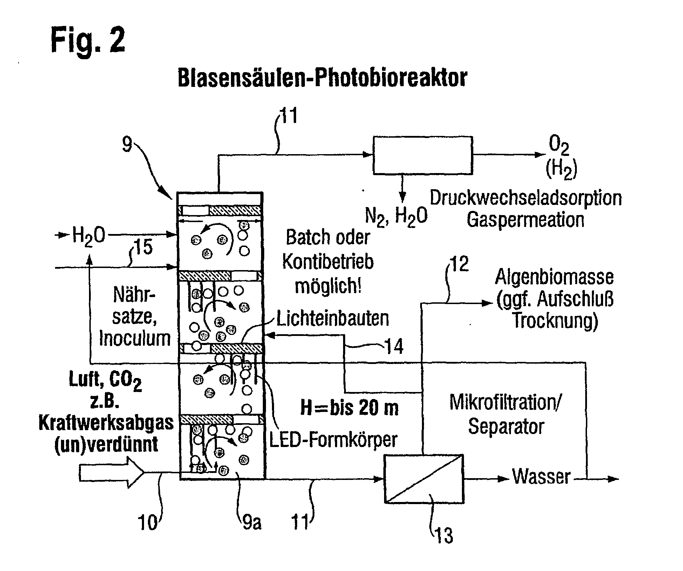

Die Erfindung wird in Figur 1 bis 3 beispielhaft erläutert:
Figur 1: Photobioreaktorelement mit LED-Silicon-Formteilen im Rohrbündel oder Schlauchbündel.
   Das Photobioreaktorelement 1 ist aus einem drucktragenden Reaktormantel 1a aufgebaut, und verfügt über ein Lichteinbauteil 1b, im Sinne eines Kolonneneinbauteils (spezieller Kolonnenboden) oder einen Lichteinbauflansch, der im Sonderfall zugleich Photobioreaktordeckel sein kann. In Reaktormantel 1a oder Photobioreaktordeckel 1b können Stutzen für Zu- und Abführungen angeordnet sein. Das Lichteinbauteil oder der Lichteinbauflansch 1b besitzt eine Durchtrittsöffnung 2 für das nächste Photobioreaktorelement (siehe Figur 2). Der Photobioreaktor ist mit einer begasten wässrigen Algensuspension 3 befüllt und steht über die Durchtrittsöffnung des darunterliegenden Photobioreaktorelements mit diesem gas- und suspensionsseitig in Verbindung. Die Gasblasen steigen dabei auf und induzieren eine Flüssigkeitsumwälzung, Durchmischung und Suspendierung der Mikroalgen.
   Das Lichteinbauteil (Lichteinbauflansch, im Sonderfall Photobioreaktordeckel) 1b enthält ein parallel geschaltetes Bündel 5 von LED-Silicon-Formteilen 6, welche in Reihe verschaltete rote LEDs (rote Punkte) 7 enthalten. Das Bündel 5 ist mittels einer Vergußdichtmasse mit dem Rohrboden des Lichteinbauteils (Lichteinbauflansches, im Sonderfall Reaktor-Abdeckung) 1b verbunden.
Figur 2: Blasensäulen-Photobioreaktor
   Figur 2 zeigt einen Photobioreaktor bestehend aus mehreren Photobioreaktorelementen gemäß Figur 1 zu einem Säulenreaktor 9 zusammengesetzt. Am unteren Ende der Reaktorsäule 9 wird der unterste Reaktorbestandteil 9a über Leitung 10 und Gasverteiler mit CO₂-haltiger Luft bzw. Kratfwerksabgas beschickt. Am oberen Ende treten über Leitung 11 das CO₂-verarmte, aufgereinigte Gas aus, bzw. können gasförmige Produkte entnommen und in nachfolgenden Verfahrensstufen abgetrennt werden. Über Leitung 12 kann wässrige Algensuspension entnommen werden. Die Algenbiomasse kann in einer Mikrofiltration/Separator-Einheit 13 als Retentat konzentriert werden. Ein Teilstrom kann zur Erhöhung der Mikroalgenkonzentration über Leitung 14 zurückgeführt werden. Das Wasser wird, gegebenenfalls nach Anreicherung mit Nährsalzen, vorzugsweise oben in den Photobioreaktor zugeführt und sofern eine Mikrofiltrations/-Separatoreinheit 13 installiert ist, als Permeat abgetrennt. Ein Teilstrom kann ggf.über Leitung 15 zurückgeführt werden. Der Säulenreaktor 9 kann batchweise, im Feedbatchbetrieb oder kontinuierlich betrieben werden.

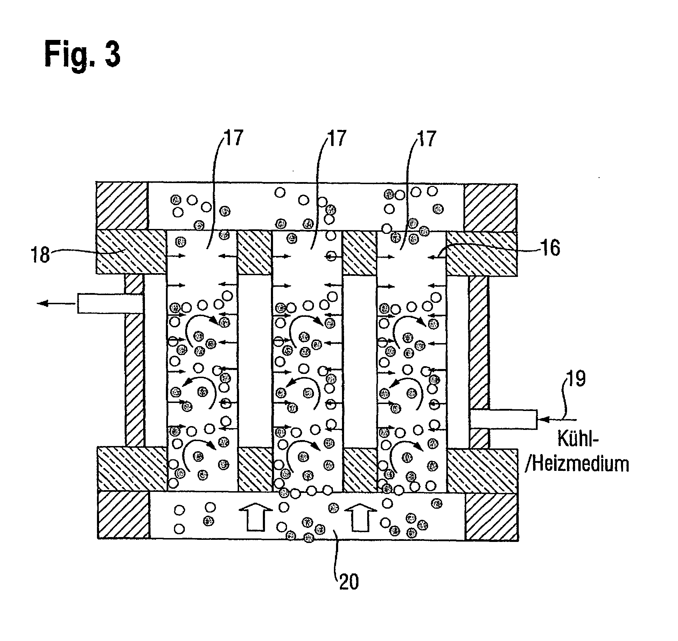

Figur 3: Blasensäulenreaktor mit parallel geschalteten LED-Silicon-Rohren
   Figur 3 zeigt einen Photobioreaktor bei welchem mehrere mit LEDs 16 bestückte, transparente Siliconrohre 17 als Rohrbündel parallel geschaltet sind, und mit einem Rahmen 18 verbunden sind. Die Siliconrohre werden mit einem Heiz/Kühl-System 19 temperiert. Mehrere solche Photoreaktoreinbauteile können als Säulenreaktor übereinander verschaltet und/oder in Rühr-Airlift- Blasensäulen- oder Schlaufenreaktoren eingebaut werden.

### Beispiel 1:

Ein geschlossener Photosyntheserohrreaktor aus Edelstahl mit einem Durchmesser von 365 mm, einer Höhe von 28 m, und mit 27 im Abstand von je 1 m übereinander angeordneten Lichteinbauten, mit je 255 nach unten stehenden LED-Silicon-Formteilen (Abmessungen (HxBxT) 965 mm x 8 mm x 13 mm), bestehend aus einem im Silicon eingebetteten LED-Band (Anschlußleistung 8,4 W; Vf= 12 V lf= 700 mA), mit einem Abstand zwischen zwei LED-Silikon-Formteilen von ca. 2 cm, wurde zur autotrophen Produktion von Algenbiomasse (Chlorella vulgaris) unter sterilen Bedingungen vollkontinuierlich über einen Zeitraum von 4 Wochen betrieben.

Der Reaktor war zur Sterilisation und Kühlung mit außen aufgeschweißten Halbrohrschlangen ausgeführt und mit Temperatur-, Druck, ph- und Standregelung ausgestattet.
Die Sterilisation erfolgte im mit Medium (Wasser + Nährsalze) gefüllten Zustand bei 121°C und Überdruck über einen Zeitraum von einer Stunde. Hierzu wurden die Halbrohrschlangen mit Dampf 6 bar durchströmt, die Dampfzufuhr wurde über die Temperatur im Reaktor geregelt. Beim Abkühlen wurde zunächst die Begasung gestartet, um das Entstehen von Unterdruck zu vermeiden.
Die Betriebstemperatur im Reaktor wurde anschließend über die Kühlwasserzufuhr in den Doppelmantel konstant geregelt und auf 27°C eingestellt. Am Reaktorkopf wurde ein leichter Überdruck von 10 mbar eingestellt. Zu-, Abluft und Wasserfeed wurden sterilgefiltert, die Algensuspension in einen sterilisierten Sammelbehälter entleert.
Der Reaktor wurde mit einer Chlorella-Vorkultur beimpft, die in Schüttelkolben hergestellt wurde.
Nach Erreichen einer Biotrockenmassekonzentration von 2 % (20g/l), wurde auf kontinuierlichen Betrieb umgestellt.
Die Verdünnungsrate wurde so eingestellt, dass sich im Reaktor eine wässrige Algensuspension (Algen: Clorella vulgaris) mit 2 Gew.-% Algenanteil stationär einstellte. Zur Wasserversorgung der Algen und zur Zuführung von Nährsalzen war dazu ein kontinuierlicher Feedwasserstrom von 8,8 1/h notwendig. Die Anlage wurde mit 20 Nm³/h eines CO2-haltigen Abgases beschickt, welches 4,5 Vol.-% CO₂, 10,2 Vol.-% O₂, 10,8 Vol.-% H₂O und 74,5 Vol.-% N₂ enthielt.
Das aus dem Reaktor austretende Abgas hatte die Zusammensetzung 0, 1 % CO₂, 16, 0 % O₂, 2,9 % H₂O und 81, 0 % N₂.
Die 2 %ige Algensuspension wurde füllstandsgeregelt kontinuierlich aus dem Reaktor entnommen. Der Reaktor wurde täglich 3x beprobt. Die Reaktorproduktivität betrug im kontinuierlichen Dauerbetrieb 0,4 kg Algentrockenmasse/m³/h.

## Patentansprüche

1. Photoreaktoren mit LED-Kunststoff-Formteilen, in denen mehrere LED-Leuchtkörper in einer Kunststoffmatrix eingeschlossen sind, als Strahlungsquellen, welche im Innern des Photoreaktors angeordnet sind, **dadurch gekennzeichnet, dass** als LED-Kunststoff-Formteile LED-Silicon-Formteile eingesetzt werden.

2. Photoreaktor nach Anspruch 1, **dadurch gekennzeichnet, dass** strahlungsemittierende Halbleiterbauelemente von organischen oder anorganischen Halbleitern als LED-Leuchtkörper eingesetzt werden.

3. Photoreaktor nach Anspruch 1 bis 2, **dadurch gekennzeichnet, dass** die LED-Silicon-Formteile mehrere LED-Leuchtkörper miteinander leitend verbunden, in Reihe und/oder parallel geschaltet enthalten.

4. Photoreaktor nach Anspruch 1 bis 3, **dadurch gekennzeichnet, dass** Lichtleiter-Formteile als strahlungsquelle neben Kunststoff-LED-Formteilen eingesetzt werden.

5. Photoreaktor nach Anspruch 4, **dadurch gekennzeichnet, dass** als Lichtleiter-Formteile solche auf der Basis von Thermoplastischen Silicon-Elastomeren eingesetzt werden.

6. Photoreaktor nach Anspruch 1 bis 5, **dadurch gekennzeichnet, dass** zur unterstützenden Lichtverteilung im Photoreaktor neben Kunststoff-LED-Formteilen und/oder Lichtleiter-Formteilen lumineszierende Silicon-Schwebekörper eingesetzt werden.

7. Photoreaktor nach Anspruch 1 bis 6, **dadurch gekennzeichnet, dass** die LED-Kunststoff-Formteile und die Lichtleiter in Form von Schläuchen vorliegen, als Bänder, als Rohre, als Platten oder in Form von Matten.

8. Photoreaktor nach Anspruch 1 bis 7, **dadurch gekennzeichnet, dass** der Photoreaktor mehrere LED-Kunststoff-Formteile, welche als Rohre, Schläuche oder Platten ausgebildet sind, enthält, und diese als Rohr-, Schlauch- oder Plattenbündel zu Lichteinbauten zusammengefasst werden.

9. Photoreaktor nach Anspruch 1 bis 8, welcher als Photobioreaktor LED-Kunststoff-Formteile in Röhren- oder Plattenform enthält, die von der Algensuspension durchströmt werden, wobei mehrere Rohre oder Platten zu Rohr- oder Plattenbündel zusammengefasst werden und eine Reaktoreinheit bilden, die im Mantelraum durch ein Kühlmedium durchströmt wird.

10. Photoreaktor nach Anspruch 1 bis 9, **dadurch gekennzeichnet, dass** die LED-Kunststoff-Formteile solche sind auf Basis von vernetzten Siliconkautschuken, Siliconhybridpolymeren und/oder Siliconharzen.

11. Photoreaktor nach Anspruch 1 bis 10, **dadurch gekennzeichnet, dass** die LED-Kunststoff-Formteile eine einzige Siliconmatrix aus thermoplastischen Elastomeren enthalten

12. Photoreaktor nach Anspruch 1 bis 11, **dadurch gekennzeichnet, dass** die LED-Kunststoff-Formteile eine innere, weiche Siliconmatrix A, welche von einer oder mehreren, härteren Siliconmatrices B umgeben wird, enthalten.

13. Photoreaktor nach Anspruch 1 bis 12, **dadurch gekennzeichnet, dass** die Kunststoffmatrix noch mit einem Topcoat C versehen ist.

14. Verfahren zur Durchführung von strahleninduzierten chemische Reaktionen in einem Photoreaktor nach Anspruch 1 bis 13.

15. Verfahren zur Produktion von Algenbiomasse mit einem Photoreaktor nach Anspruch 1 bis 13.

16. Verfahren nach Anspruch 15 zur autotrophen und heterotrophen Produktion von Wertstoffen mittels Algenbiomasse.

17. Verfahren zur Entfernung von CO₂ aus Kraftwerks- oder Industrieabgasen mit einem Photoreaktor nach Anspruch 1 bis 13.

18. Verfahren zur Produktion von Wasserstoff oder anderen gasförmigen Stoffwechselprodukten mit Mikroalgen oder Mikroorganismen, die eine Energiezufuhr in Form von Licht erfordern, mit einem Photoreaktor nach Anspruch 1 bis 13.

19. Verwendung der im Photoreaktor nach Anspruch 1 bis 13 hergestellten Algenbiomasse als Energierohstoff, Chemierohstoff, Nahrungsmittel, in kosmetischen und medizinischen Anwendungen.

## Claims

1. Photoreactors comprising LED plastic molded parts in which a plurality of LED luminous bodies are enclosed in a plastic matrix, as radiation sources which are arranged inside the photoreactor, **characterized in that** LED silicone molded parts are used as the LED plastic molded parts.

2. Photoreactor according to Claim 1, **characterized in that** radiation-emitting semiconductor components comprising organic or inorganic semiconductors are used as the LED luminous bodies.

3. Photoreactor according to Claims 1 to 2, **characterized in that** the LED silicone molded parts contain a plurality of LED luminous bodies joined together conductively and connected in series and/or parallel.

4. Photoreactor according to Claims 1 to 3, **characterized in that** light-guide molded parts are used as a radiation source besides plastic LED molded parts.

5. Photoreactor according to Claim 4, **characterized in that** the light-guide molded parts used are ones based on thermoplastic silicone elastomers.

6. Photoreactor according to Claims 1 to 5, **characterized in that** luminescent silicone floating bodies are used for supportive light distribution in the photoreactor, besides plastic LED molded parts and/or light-guide molded parts.

7. Photoreactor according to Claims 1 to 6, **characterized in that** the LED plastic molded parts and the light guides are provided in the form of pipes, as strips, as tubes, as plates or in the form of mats.

8. Photoreactor according to Claims 1 to 7, **characterized in that** the photoreactor contains a plurality of LED plastic molded parts, which are formed as tubes, pipes or plates, and these are combined as pipe, tube or plate bundles to form lighting inserts.

9. Photoreactor according to Claims 1 to 8, which as a photobioreactor contains LED plastic molded parts in tube or plate form, through which the alga suspension flows, a plurality of tubes or plates being combined to form tube or plate bundles and forming a reactor unit through which a cooling medium flows in the shell space.

10. Photoreactor according to Claims 1 to 9, **characterized in that** the LED plastic molded parts are ones based on crosslinked silicone rubbers, silicone hybrid polymers and/or silicone resins.

11. Photoreactor according to Claims 1 to 10, **characterized in that** the LED plastic molded parts contain a single silicone matrix of thermoplastic elastomers.

12. Photoreactor according to Claims 1 to 11, **characterized in that** the LED plastic molded parts contain a soft inner silicone matrix A, which is enclosed by one or more harder silicone matrices B.

13. Photoreactor according to Claims 1 to 12, **characterized in that** the plastic matrix is also provided with a topcoat C.

14. Method for carrying out radiation-induced chemical reactions in a photoreactor according to Claims 1 to 13.

15. Method for producing alga biomass using a photoreactor according to Claims 1 to 13.

16. Method according to Claim 15 for the autotrophic and heterotrophic production of valuable substances by means of alga biomass.

17. Method for removing CO₂ from power plant or industrial waste gases using a photoreactor according to Claims 1 to 13.

18. Method for producing hydrogen or other gaseous metabolic products using microalgae or microorganisms, which require an energy supply in the form of light, using a photoreactor according to Claims 1 to 13.

19. Use of the alga biomass produced in the photoreactor according to Claims 1 to 13 as an energy raw material, chemical raw material, foodstuff, in cosmetic and medical applications.

## Revendications

1. Photoréacteurs à composants LED moulés en matière synthétique, dans lesquels plusieurs corps lumineux LED qui servent de sources de rayonnement sont incorporés dans une matrice en matière synthétique et sont disposés à l'intérieur du photoréacteur,
**caractérisés en ce que**
comme composants LED moulés en matière synthétique, on utilise des composants LED moulés en silicone.

2. Photoréacteur selon la revendication 1, **caractérisé en ce que** des éléments semi-conducteurs émettant un rayonnement, constitués de semi-conducteurs organiques ou minéraux, sont utilisés comme corps lumineux LED.

3. Photoréacteur selon les revendications 1 et 2, **caractérisé en ce que** les composants LED moulés en silicone contiennent plusieurs corps lumineux LED reliés les uns aux autres de manière conductrice et raccordés en série et/ou en parallèle.

4. Photoréacteur selon les revendications 1 à 3, **caractérisé en ce que** des composants moulés des conducteurs de lumière sont utilisés comme source de rayonnement en plus de composants LED moulés en matière synthétique.

5. Photoréacteur selon la revendication 4, **caractérisé en ce que** comme composants moulés de conducteurs de lumière, on utilise des composants moulés à base d'élastomères thermoplastiques de silicone.

6. Photoréacteur selon les revendications 1 à 5, **caractérisé en ce que** pour soutenir la répartition de lumière dans le photoréacteur, en plus de composants LED moulés en matière synthétique et/ou de composants moulés de conducteurs de lumière, on utilise des corps flottants luminescents en silicone.

7. Photoréacteur selon les revendications 1 à 6, **caractérisé en ce que** les composants LED moulés en matière synthétique et les conducteurs de lumière présentent la forme de tuyaux flexibles, de bandes, de tubes, de plaques ou de tapis.

8. Photoréacteur selon les revendications 1 à 7, **caractérisé en ce que** le photoréacteur contient plusieurs composants LED moulés en matière synthétique configurés sous la forme de tubes, de tuyaux flexibles ou de plaques, qui sont rassemblés sous la forme de faisceaux de tubes, de tuyaux flexibles ou de plaques pour former des garnitures lumineuses.

9. Photoréacteur selon les revendications 1 à 8, qui, comme photobioréacteur, contient des composants LED moulés en matière synthétique en forme de tubes ou de plaques traversés par la suspension d'algues, plusieurs tubes ou plaques étant rassemblés pour former des faisceaux de tubes ou de plaques et formant une unité de réacteur dont l'espace d'enveloppe est traversé par un fluide de refroidissement.

10. Photoréacteur selon les revendications 1 à 9, **caractérisé en ce que** les composants LED moulés en matière synthétique sont des composants moulés à base de caoutchoucs au silicone réticulés, de polymères hybrides de silicone et/ou de résines de silicone.

11. Photoréacteur selon les revendications 1 à 10, **caractérisé en ce que** les composants LED moulés en matière synthétique contiennent une unique matrice de silicone en élastomères thermoplastiques.

12. Photoréacteur selon les revendications 1 à 11, **caractérisé en ce que** les composants LED moulés en matière synthétique contiennent une matrice intérieure malléable A en silicone entourée par une ou plusieurs matrices B plus dures en silicone.

13. Photoréacteur selon les revendications 1 à 12, **caractérisé en ce que** la matrice de matière synthétique est de plus dotée d'une couche de recouvrement C.

14. Procédé d'exécution de réactions chimiques induites par rayonnement dans un photoréacteur selon les revendications 1 à 13.

15. Procédé de production de biomasse d'algues à l'aide d'un photoréacteur selon les revendications 1 à 13.

16. Procédé selon la revendication 15 de production autotrophe ou hétérotrophe de substances de valeur à partir de biomasse d'algues.

17. Procédé d'élimination de CO₂ de gaz d'effluent de centrales électriques ou de l'industrie à l'aide d'un photoréacteur selon les revendications 1 à 13.

18. Procédé de production d'hydrogène ou d'autres produits gazeux du métabolisme à l'aide de microalgues ou de microorganismes qui nécessitent un apport d'énergie sous forme de lumière, à l'aide d'un photoréacteur selon les revendications 1 à 13.

19. Utilisation de la biomasse d'algues préparée dans le photoréacteur selon les revendications 1 à 13 comme matière première énergétique, matière première de la chimie, produit alimentaire, dans des applications cosmétiques et médicales.
